# FASCICULE DE BREVET EUROPEEN

(11) **EP 1 837 811 B1**
(45) Date de publication et mention de la délivrance du brevet: **02.12.2009**
(21) Numéro de dépôt: 07005218.8
(22) Date de dépôt: 14.03.2007
(51) Int. Cl.: G06K 19/077, A61J 1/03

(54) **Procédé de fabrication d'une étiquette éléctronique RFID**
Verfahren zur Herstellung eines elektronischen RFID-Etiketts
Method of manufacturing an RFID electronic tag

(30) Priorité: 21.03.2006 FR 0602463
(43) Date de publication de la demande: 26.09.2007
(73) Titulaire: STMICROELECTRONICS SA, 92120 Montrouge (FR); STMicroelectronics, Inc., Carrollton Texas 75006 (US)
(72) Inventeur: Fidelis, Sylvain, Austin, TX 78729 (US); Rizzo, Pierre, 13100 Aix en provence (FR)
(74) Mandataire: Marchand, André

(56) Documents cités:
- EP-A- 1 758 050
- WO-A-2006/002667
- US-A1- 2004 005 418
- US-A1- 2006 038 286

## Description

La présente invention concerne la réalisation d'étiquettes électroniques RFID (Radio Frequency Identification).

Les étiquettes électroniques RFID sont réalisées au moyen de circuits intégrés sans contact passifs, ou transpondeurs, qui peuvent être du type à couplage électrique ou du type à couplage inductif.

Les circuits intégrés sans contact à couplage électrique, ou circuits intégrés sans contact UHF, sont alimentés électriquement par un champ électrique UHF oscillant à plusieurs centaines de MHz et émettent des données par modulation du taux de réflexion de leur circuit d'antenne (technique appelée "backscattering"). De tels circuits intégrés sont par exemple décrits par la spécification industrielle EPCTM-GEN2 ("Radio-Frequency Identity Protocols Class-1 Generation-2 - UHF RFID Protocol for Communications at 860 MHz - 960 MHz") en cours de normalisation. Ils sont généralement utilisés dans les applications dites à longue portée ("long range") dans lesquelles la distance entre le circuit intégré et une station d'émission/réception de données émettant le champ électrique, appelée communément un lecteur, peut atteindre plusieurs mètres.

Les circuits intégrés sans contact à couplage inductif, ou circuits intégrés sans contact RF, émettent des données par modulation de charge et sont alimentés par couplage inductif en présence d'un champ magnétique dont la fréquence est généralement de l'ordre de la dizaine de MHz. De tels circuits intégrés sont par exemple décrits par les normes ISO/IEC 14443A/B, ISO/IEC 15693 qui prévoient une fréquence de travail de 13,56 MHz.

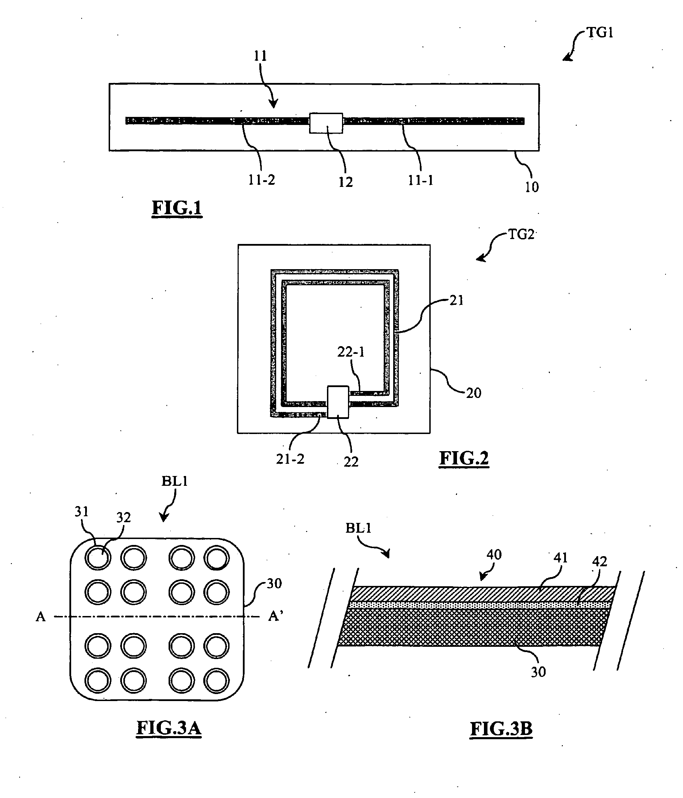

La figure 1 représente schématiquement une étiquette électronique TG1 de type UHF. L'étiquette électronique TG1 comprend un substrat 10, une antenne dipolaire UHF 11 comprenant deux brins d'antenne 11-1, 11-2, et un circuit intégré UHF 12 ayant deux plages de contact connectées aux brins d'antenne 11-1, 11-2.

La figure 2 représente schématiquement une étiquette électronique TG2 de type RF. L'étiquette électronique TG2 comprend un substrat 20, une antenne 21 en forme de bobine, comprenant par exemple deux enroulements, et un circuit intégré RF 22 ayant deux plages de contact connectées aux extrémités 21-1, 21-2 de la bobine d'antenne.

Le substrat 10, 20 peut être une simple feuille de matière plastique souple (polyamide, polyester...) ayant une face arrière autocollante, voire un simple substrat de papier. L'antenne dipolaire UHF 11 ou la bobine d'antenne RF 21 peut être réalisée par gravure d'une couche de métal, par collage sur le support d'un motif métallique prédécoupé ("leadframe"), par dépôt d'une encre conductrice (sérigraphie ou jet d'encre), etc. Il est également connu de réaliser l'antenne 11 ou 21 directement sur le produit de destination, par dépôt d'encre conductrice sur une face du produit, laquelle forme alors le substrat de l'étiquette.

De telles étiquettes électroniques UHF ou RF sont couramment utilisées pour l'identification et/ou l'authentification des produits sur lesquels elles sont apposées. Le circuit intégré sans contact comprend le plus souvent une mémoire effaçable et programmable électriquement permettant d'enregistrer, outre des données d'identification, des données d'application, des données commerciales, des données de traçage, etc. Les applications offertes sont ainsi très nombreuses et comprennent l'authentification de produits (lutte contre la contrefaçon ou la contrebande), la gestion des stocks, la gestion logistique (suivi des produits pendant le transport), etc.

En raison du prix de revient de plus en plus faible des circuits intégrés sans contact, les étiquettes électroniques sont destinées à être apposées sur un nombre de plus en plus importants de produits. Pour satisfaire des exigences de respect de la vie privée du consommateur, il a été proposé de réaliser une étiquette électronique pouvant être détruite par l'acheteur après achat du produit. Une étiquette électronique étant généralement accessible en lecture et en écriture, elle pourrait en effet être utilisée frauduleusement pour identifier ou suivre secrètement non seulement le produit revêtu de l'étiquette mais également la personne ayant fait l'acquisition du produit.

Plutôt qu'une mise hors service de l'étiquette au moyen de la commande classique "KILL", difficile à vérifier par le consommateur final, une destruction physique du circuit d'antenne est considérée comme la solution la plus rassurante pour le consommateur, celui-ci pouvant s'assurer visuellement que l'étiquette est hors d'usage.

Le document intitulé "Disabling RFID Tags with visible confirmation - Clipped tags are silenced" de Gunter Karjoth (IBM Zurich Research Laboratory Ruschlikon) et de Paul Moskowitz (IBM T.J. Watson Research Center Hawthorne) du 31 août 2005, Computer Science (Références du document RC23710(W0508-164)), décrit diverses solutions permettant à l'utilisateur de mettre une étiquette électronique hors service, à savoir :
1) la réalisation de tout ou partie de l'antenne sur un matériau à gratter ("scratch-off material") devant ainsi être gratté par l'utilisateur pour détruire l'antenne,
2) la réalisation de tout ou partie de l'antenne sur un support prédécoupé devant être retiré par l'utilisateur, une languette d'arrachement étant prévue à cet effet,
3) la réalisation de tout ou partie de l'antenne sur une couche "à peler" ("peel-off layer") que l'utilisateur doit également retirer pour mettre l'antenne hors service, toujours au moyen d'une languette d'arrachement.

Ces diverses solutions visant une destruction de l'antenne complexifient toutefois la structure des étiquettes électroniques "prêtes à être posées" et augmentent par conséquent leur prix de revient.

Le brevet US 6,574,166 décrit par ailleurs une méthode permettant de détecter et de gérer la consommation de comprimés agencés dans un emballage de type "blister". Selon le mode de réalisation représenté en figure 2 de ce brevet, un réseau de conducteurs électriques (108, 210) est prévu dans la feuille d'emballage du blister (feuille recouvrant les alvéoles du blister). Le réseau de conducteurs est pris en sandwich entre deux feuilles plastiques (208, 212) électriquement isolantes au-dessus desquelles s'étend un film d'aluminium (104), les deux feuilles plastique et la feuille d'aluminium formant la feuille d'emballage. Lorsque l'utilisateur ouvre une alvéole du blister pour expulser un comprimé, il rompt l'un des conducteurs. La rupture est détectée comme un circuit ouvert par un circuit électronique connecté au réseau de conducteurs.

Dans le même ordre d'idée, la société ABR Pharma commercialise un blister Med-ic^{®} dit "intelligent" (www.abrpharma.com/PDF/blister.pdf), comportant un réseau de conducteurs quadrillé réalisé par dépôt d'encres conductrices à base d'argent et de graphite sur un substrat de papier. Le réseau de conducteurs est collé sur la face avant en aluminium du blister et est conformé de telle sorte que l'un des conducteurs est détruit lorsque le patient extrait un comprimé d'une alvéole. La rupture du conducteur est détectée par un circuit intégré RFID 13,56 MHz qui retransmet à un lecteur les données d'ouverture des alvéoles.

Cette technique de détection de la prise de comprimés est basée sur la destruction de conducteurs incorporés dans la feuille d'emballage du blister mais n'est pas destructive pour le circuit d'antenne, puisque le circuit intégré RFID relié au réseau de conducteurs doit pouvoir continuer à échanger des données avec un lecteur afin d'envoyer des informations concernant le nombre de comprimés restant dans le blister.

Une première idée de l'invention, ayant un lien de parenté avec l'enseignement du brevet US 6,574,166 mais ne découlant pas de façon évidente de celui-ci, est de réaliser une antenne d'étiquette RF ou UHF dans l'emballage même d'un produit, de manière que l'étiquette soit détruite au moment de l'ouverture du produit, ou qu'elle soit, à tout le moins, "jetée à la poubelle" lorsque l'emballage est retiré du produit.

La technique divulguée par US 6,574,166, pour réaliser les conducteurs de détection d'ouverture des alvéoles du blister, nécessite de prévoir deux couches de matériau isolant pour éviter que la couche supérieure d'aluminium de la feuille d'emballage du blister ne court-circuite le réseau de conducteurs. Cette technique est coûteuse et ne peut être retenue pour réaliser une antenne à faible coût intégrée dans une feuille d'emballage, le prix de revient des étiquettes "prêtes à poser" restant plus avantageux.

La présente invention vise un procédé permettant de réaliser à moindre coût une étiquette électronique qui puisse optionnellement être détruite lors de l'ouverture d'un emballage.

US 2006/038286 divulgue une feuille d'emballage, de conditionnement ou de transport d'un produit comprenant un film électriquement conducteur. Ce film conducteur comporte des zones dépourvues de matière conductrice délimitant dans le film électriquement conducteur au moins un motif d'antenne formant une antenne pour étiquette RFID. Les caractéristiques dans le préambule de la revendication 1 sont connues de ce document.

US 2004/005418 divulgue une feuille d'emballage ayant une antenne formée par une étape de gravure.

WO 2006/002667 divulgue un emballage de type blister comprenant une plaquette ayant des alvéoles. Une feuille d'emballage comprenant un film support électriquement isolant ferme les alvéoles. Un film métallique obtenu par condensation de vapeur est déposé sur le film support électriquement isolant et une puce est déposée sur la feuille d'emballage et est connectée à une antenne créée sur cette feuille.

EP-A-1 758 050 (opposable au titre de l'article 54(3) CBE) divulgue une feuille 1 d'emballage, de conditionnement ou de transport d'un produit comprenant un film électriquement conducteur. Ce film conducteur comporte des zones (autour d'une île) dépourvues de matière conductrice délimitant dans le film conducteur au moins un motif d'antenne formant une antenne pour étiquette RFID.

A cet effet, la présente invention propose d'utiliser les parties métalliques ou électriquement conductrices d'un matériau d'emballage, de conditionnement ou de transport d'un produit, pour réaliser une antenne RF ou UHF, en retirant de la matière métallique ou de la matière conductrice de manière à faire apparaître un motif d'antenne.

La présente invention se fonde notamment sur l'idée d'utiliser le film métallique d'une feuille d'emballage de blister pour former une antenne RF ou UHF, en retirant une partie de la matière métallique de manière à faire apparaître un motif d'antenne.

Selon l'invention, cette idée est généralisée à tout type de produit autre qu'un blister pour lequel une feuille d'emballage, de conditionnement ou de transport comportant un film métallique est utilisée. Le motif d'antenne est formé par retrait de celle-ci au moyen d'un procédé mécanique (abrasion, découpe...).

Ainsi, la présente invention prévoit un procédé de fabrication d'une étiquette électronique destinée à être apposée sur un produit selon la revendication 1.

Selon un mode de réalisation, la feuille d'emballage, de conditionnement ou de transport comprend au moins un film support électriquement isolant recouvert en tout ou en partie par le film conducteur.

Selon un mode de réalisation, le produit est une plaquette de type blister et la feuille d'emballage, de conditionnement ou de transport est appliquée sur une face de la plaquette avec le film conducteur orienté vers l'extérieur.

Selon un mode de réalisation, le film conducteur est un film métallique.

L'invention concerne également un procédé de fabrication d'un emballage de type blister, selon la revendication 6.

Selon un mode de réalisation, le procédé comprend une étape consistant à connecter à l'antenne une puce en matériau semi-conducteur pour former une étiquette électronique.

Ces objets, caractéristiques et avantages ainsi que d'autres de la présente invention seront exposés plus en détail dans la description suivante du procédé selon l'invention et d'exemples d'antennes réalisées selon ce procédé, faite à titre non limitatif en relation avec les figures jointes parmi lesquelles :
- les figures 1 et 2 précédemment décrites représentent respectivement des étiquettes électroniques RF et UHF classiques,
- les figures 3A et 3B représentent un blister de type classique, respectivement par une vue de dessus et une vue en coupe,
- la figure 4A est une vue de dessus d'un blister comprenant une antenne UHF,
- la figure 4B est une vue de dessus du blister de la figure 4A après montage et connexion d'un circuit intégré sans contact sur l'antenne,
- les figures 5 et 6 sont des vues en coupe représentant deux modes de montage et de connexion du circuit intégré sur l'antenne de la figure 4A,
- la figure 7A est une vue de dessus d'une plaque de blister en formation illustrant une étape de fabrication d'un blister selon l'invention,
- la figures 7B est une vue en coupe d'une partie du blister de la figure 7A,
- la figure 7C est une vue en coupe d'une partie du blister de la figure 7A après application d'une feuille d'emballage sur le blister,
- les figures 8A, 8B sont des vues en coupe du blister de la figure 7C illustrant un premier procédé de fabrication d'une antenne selon l'invention,
- la figure 9A est une vue de dessus d'un blister comprenant une bobine d'antenne,
- la figure 9B est une vue grossie de la bobine d'antenne de la figure 9A,
- la figure 10 est une vue de face d'un paquet de cigarette comprenant une bobine d'antenne conforme à celle de la figure 9B,
- la figure 11 illustre schématiquement un second procédé de fabrication d'une antenne qui n'est pas selon l'invention,
- la figure 12 représente une feuille d'emballage après mise en oeuvre du second procédé et conditionnée sous forme de rouleau,
- les figures 13A à 13D sont des vues en coupe de la feuille d'emballage au cours d'étapes de formation de l'antenne suivant le procédé illustré en figure 11,
- les figures 14A, 14B représentent une feuille d'emballage conditionnée en rouleau respectivement avant et après mise en oeuvre d'une variante du procédé illustré en figure 11,
- la figure 15 est une vue de face d'un paquet de cigarette emballé dans la feuille d'emballage de la figure 14B, et
- la figure 16 est un schéma-bloc représentant l'architecture d'un circuit intégré sans contact classique.

Comme indiqué plus haut, la présente invention se fonde sur l'idée simple mais non moins inventive que le film d'aluminium des feuilles d'emballage comprenant un tel film d'aluminium, notamment celles utilisées pour recouvrir les blisters, peut être utilisé pour former une antenne RF ou UHF en retirant de la matière métallique en des zones appropriées de manière à faire apparaître un motif d'antenne.

La figure 3A représente un blister classique BL1 vu de dessus avant sa fermeture. A ce stade, le blister est une plaquette thermoformée 30 comprenant des alvéoles 31 recevant des comprimés 32. Après insertion des comprimés 32, la plaquette est refermée au moyen d'une feuille à film métallique qui sera désignée ici une "feuille d'emballage", l'invention ne faisant pas la différence entre les feuilles d'emballage utilisées pour empaqueter intégralement un produit et les feuilles d'emballage appliquées sur une face ou une partie d'un produit pour assurer sa fermeture.

La feuille d'emballage est représentée en coupe sur la figure 3B, faite suivant un axe de coupe AA' représenté en figure 3A, et est désignée par la référence 40. Elle comporte un film d'aluminium 41 et un film plastique 42, par exemple du vinyle copolymère, qui permet de fixer le film d'aluminium 41 sur la plaquette 30 et se trouve ainsi en contact avec les comprimés. La fixation de la feuille 40 sur la plaquette 30 est généralement faite à chaud, en faisant fondre le film 42 pour qu'il adhère à la plaquette 30 (thermosoudure).

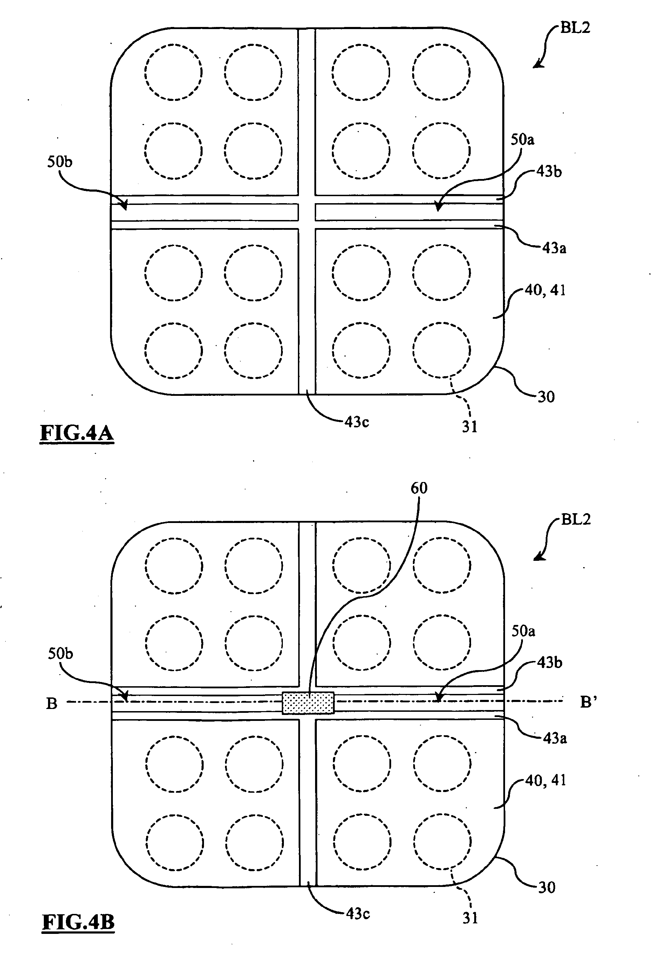

La figure 4A est une vue de dessus d'un blister BL2. Le blister comprend classiquement la plaquette 30 déjà décrite et ses alvéoles thermoformées 31 (en traits pointillés) recouvertes par la feuille 40, le film d'aluminium 41 s'étendant sur la face avant du blister. Le film 41 comprend des zones dépourvues de matière métallique, ici deux zones 43a, 43b rectilignes et parallèles passant par le centre du blister et une zone 43c rectiligne perpendiculaire aux zones 43a, 43b et passant également par le centre du blister. Les zones 43a, 43b, 43c et leurs intersections font apparaître à la surface du blister deux bandes métalliques 50a, 50b isolées électriquement du reste du film 41. Ces deux bandes métalliques forment les deux brins d'antenne d'une antenne dipolaire ou antenne UHF, utilisable pour réaliser une étiquette électronique.

L'étiquette électronique est réalisée comme représenté en figure 4B, en disposant sur le film 41 un circuit intégré sans contact 60 se présentant sous forme de puce de semi-conducteur ayant des plages de contact connectées aux brins d'antenne 50a, 50b.

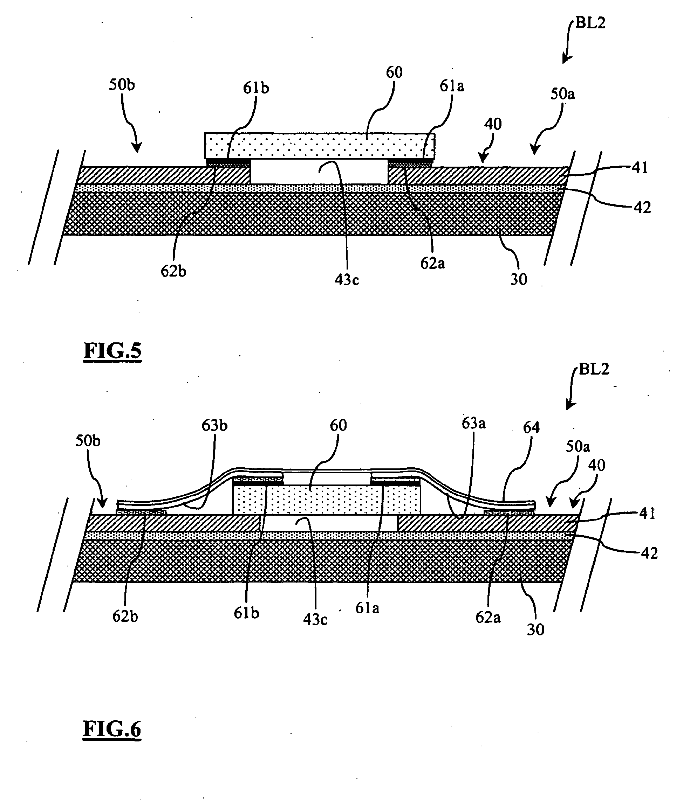

Les figures 5 et 6 sont des vues en coupe du blister selon un axe BB' représenté en figure 4B, et représentent deux exemples de montage de la puce 60.

Sur la figure 5, la puce 60 est montée selon la technique "flip-chip" (puce retournée) et présente deux plages de contact 61a, 61b qui sont collées sur les extrémités proximales des brins d'antenne 50a, 50b au moyen d'une colle conductrice 62a, 62b. Une telle colle conductrice est couramment utilisée en microélectronique et comprend généralement un polymère à charge métallique.

Sur la figure 6, on a préalablement réalisé un ensemble prêt à être monté sur l'antenne, appelé un "interposeur", en soudant sur les plages de contact 61a, 61b de la puce 60 des rectangles métalliques 63a, 63b prédécoupés sur bande métallique (technique dite "leadframe"), formant des sortes de pattes électriques. Le matériau constituant les rectangles métalliques est compatible avec le film métallique utilisé pour réaliser l'antenne selon l'invention, et est donc ici, de préférence, de l'aluminium. La face arrière de la puce 60 est recouverte d'un matériau électriquement isolant et repose sur les extrémités proximales des brins d'antenne 50a, 50b, les plages de contact 61a, 61b étant orientées vers le haut. Les extrémités distales des rectangles métalliques 63a, 63b sont rabattues et collées sur les extrémités proximales des brins d'antenne 50a, 50b au moyen d'une colle conductrice 62a, 62b. La face supérieure de l'interposeur est recouverte d'une couche protectrice 64 électriquement isolante.

On décrira maintenant deux procédés permettant d'enlever de la matière métallique dans le film d'aluminium 41 pour faire apparaître une antenne telle que celle décrite plus haut. Le premier procédé est un procédé de retrait de matière par action mécanique sur le film 41 et est mis en oeuvre après fermeture du blister (i.e. après application de la feuille d'emballage 40 sur le blister). Le second procédé est mis en oeuvre par action chimique sur la feuille d'emballage 40 avant la fermeture du blister.

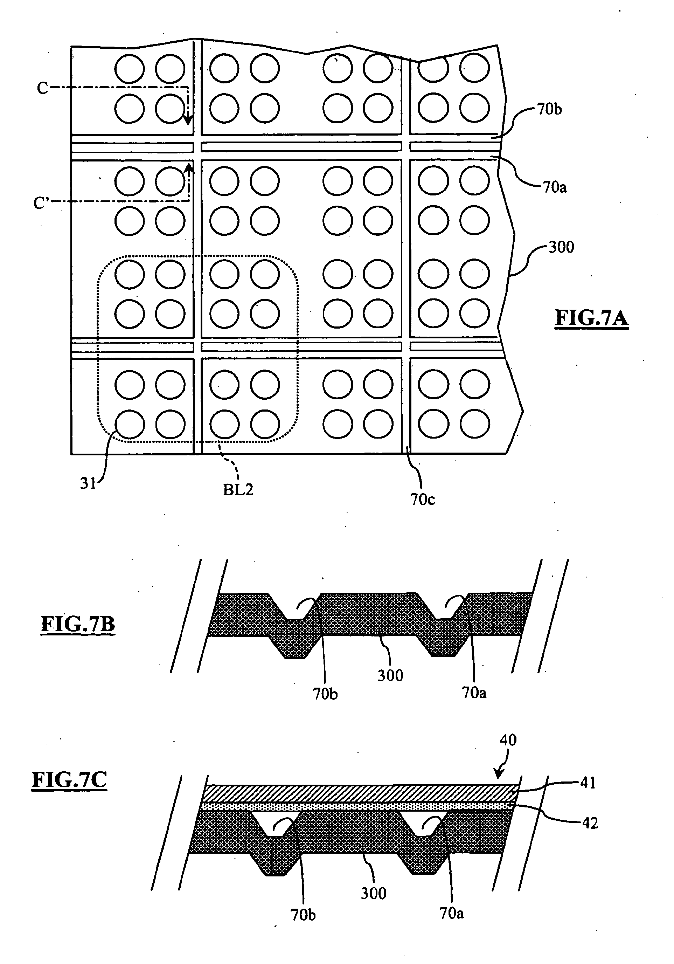

Comme illustré sur la figure 7A, le premier procédé comprend une étape préliminaire de formation de gorges ou sillons 70a, 70b, 70c à la surface d'une plaque de blister. Le blister BL2 représenté sur cette figure est fabriqué collectivement à partir d'une grande plaque 300 de matière plastique qui est thermoformée de manière à former simultanément les sillons 70a, 70b, 70c et les alvéoles de plusieurs blisters, qui sont individualisés par découpe de la plaquette 300 en fin de processus, après insertion des comprimés, fermeture de la plaque 300 et réalisation d'une antenne sur chaque blister. Les sillons 70a, 70b, 70c sont agencés de manière à s'étendre sous les zones où la matière métallique doit être retirée. Ils correspondent donc ici aux zones 43a, 43b, 43c dépourvues de matière métallique représentées en figures 4A, 4B, les sillons 70a, 70b étant rectilignes et parallèles et le sillon 70c rectiligne et perpendiculaire aux sillons 70a, 70b. Les sillons 70a, 70b, 70c sont réalisés sur la plaque 300 selon un motif répétitif (en fonction du nombre de blisters individuels à réaliser) et traversent toute la surface de la plaque.

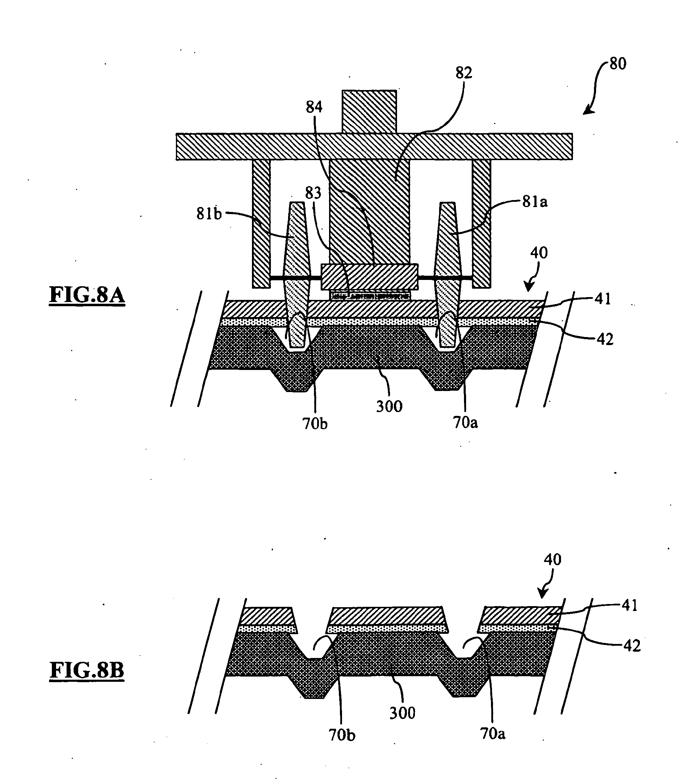

La figure 7B est une vue en coupe de la plaque 300 selon un axe CC' représenté en figure 7A, qui représente schématiquement la forme des sillons 70a, 70b. La figure 7C représente les mêmes sillons après soudure de la feuille d'emballage 40 sur la plaque 300. Les sillons 70a, 70b s'étendent sous la feuille d'emballage 40 et vont faciliter le retrait de la matière métallique au cours d'une étape illustrée sur la figure 8A.

Sur la figure 8A, le retrait de matière métallique est effectué au moyen d'un outil 80 comprenant deux molettes 81a, 81b de découpe ou d'abrasion permettant de réaliser les zones 43a, 43b dépourvues de matière métallique (figure 4A). Un outil similaire mais ne comprenant qu'une seule molette de découpe ou d'abrasion (non représenté) est utilisé pour réaliser la zone 43c. L'outil 80 comporte un sabot 82 dont la partie inférieure est équipée d'une semelle glissante 83. La semelle 83 vient s'appuyer sur le film d'aluminium 41 de la feuille 40 et permet à l'outil de glisser le long de la feuille pendant la découpe ou l'abrasion de matière. Un moteur électrique 84 peut être prévu pour entraîner les molettes en rotation, notamment si celles-ci sont des molettes abrasives. Les molettes présentent un profil effilé dans un plan perpendiculaire à leur axe de rotation et pénètrent dans les sillons 70a, 70b sans toutefois toucher le fond des sillons. La figure 8B représente en coupe l'aspect de la feuille d'emballage 40 après formation simultanée des zones 43a, 43b au-dessus des sillons 70a, 70b.

La présente invention n'est pas limitée à la réalisation d'une antenne dipolaire sur un emballage de type blister. De façon générale, le film métallique d'une feuille d'emballage peut être utilisé pour réaliser toutes sortes de formes d'antennes et une feuille d'emballage traitée conformément à l'invention peut être utilisée pour l'emballage de divers produits. Le film métallique n'est pas nécessairement en aluminium et peut comprendre tout type de métal ou alliage conducteur ayant des caractéristiques électriques satisfaisantes pour réaliser des antennes. Le film métallique peut présenter une épaisseur plus ou moins importante selon les propriétés électriques souhaitées de l'antenne, la fréquence de travail, la distance de communication sans contact visée, etc.

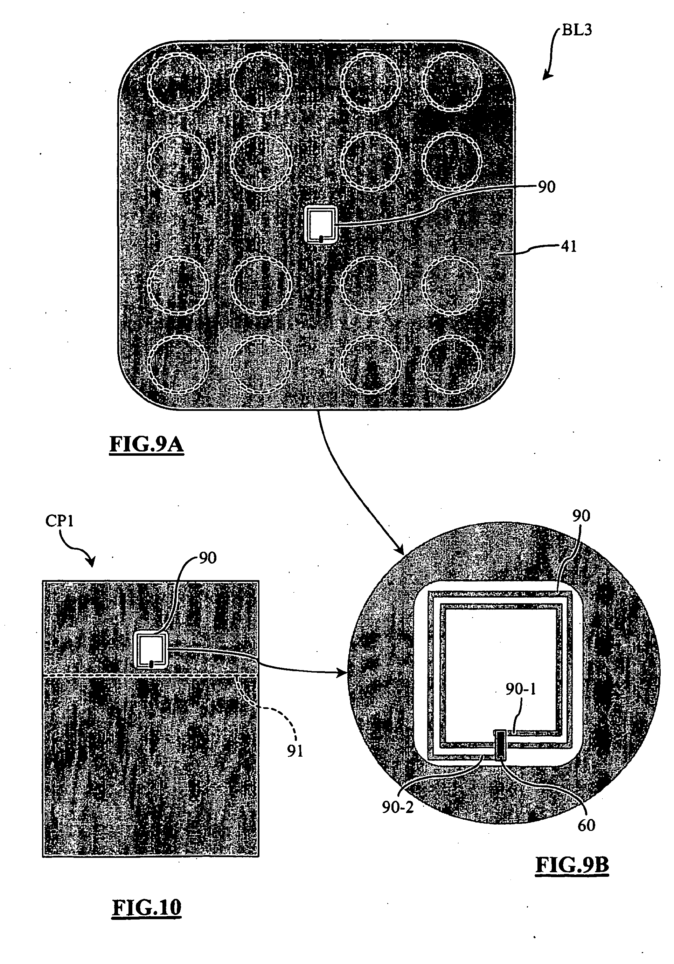

A titre d'exemple, la figure 9A représente un blister BL3 dont le film d'aluminium 41 présente un motif d'antenne en forme de bobine 90, formé par retrait de matière métallique conformément à l'invention. Comme cela apparaît plus clairement en figure 9B, un circuit intégré sans contact sous forme de puce 60 de semi-conducteur est monté et est connecté à la bobine d'antenne 90 pour former une étiquette électronique. Le montage de la puce 60 sur l'antenne 90 est effectué comme décrit plus haut en relation avec les figures 5 et 6 (flip chip ou interposeur), les plages de contact de la puce 60 étant ici reliées électriquement aux deux extrémités 90-1, 90-2 de la bobine d'antenne 90.

Sur la figure 10, la même bobine d'antenne 90 est réalisée sur le film métallique d'une feuille d'emballage d'un paquet de cigarettes CP1. Une ligne prédécoupée 91 est prévue dans la feuille d'emballage pour permettre l'ouverture du paquet. Cette ligne prédécoupée passe ici sous la zone où se trouve la bobine d'antenne 90, mais peut également être agencée de manière à traverser la bobine d'antenne 90 afin que l'ouverture du paquet entraîne sa destruction. A cet effet, des points de perçage de la feuille d'emballage, formant la ligne prédécoupée 90, doivent être suffisamment petits pour ne pas empêcher la bobine d'antenne 90 d'être opérationnelle. Plus particulièrement, ils doivent être d'un diamètre inférieur à la largeur du conducteur formant la bobine d'antenne 90 (soit la distance entre deux zones dépourvues de matière métallique).

La bobine d'antenne 90 est de préférence réalisée par gravure chimique de la feuille d'emballage avant emballage du produit, au moyen du second procédé qui n'est pas selon l'invention.

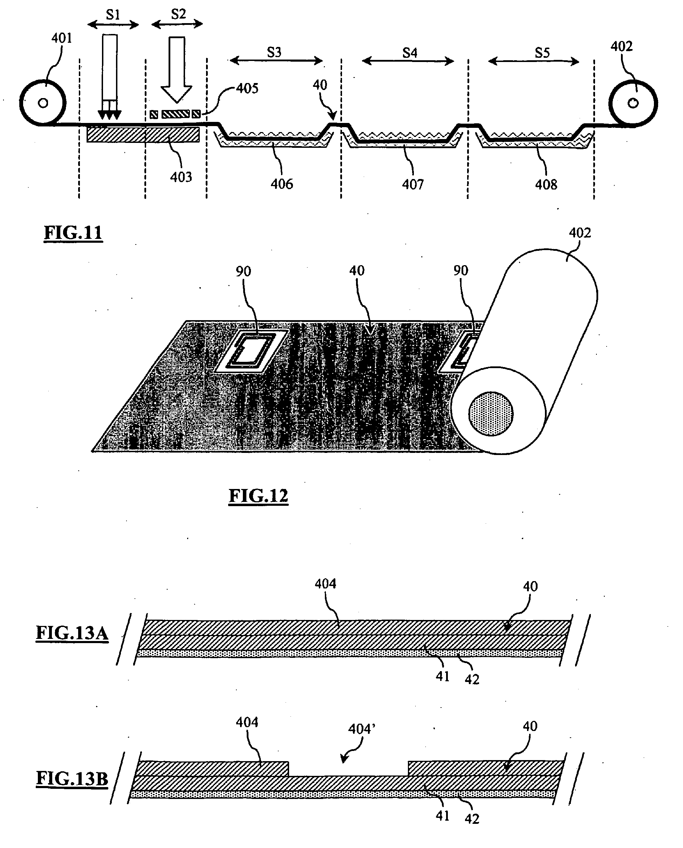

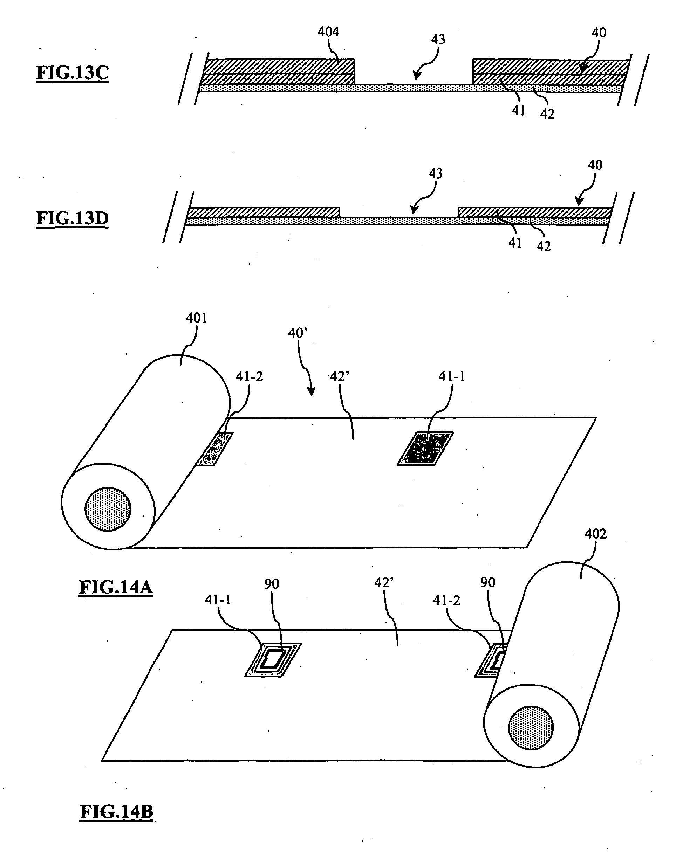

Ce procédé est illustré sur les figures 11, 13A à 13D. La feuille d'emballage 40 est préparée sous forme de rouleau 401 et le processus de gravure est mis en oeuvre en déroulant la feuille 40 sur une certaine longueur et en formant progressivement à son extrémité un "rouleau final" 402, le rouleau 401 étant ainsi le "rouleau initial". Cinq étapes de traitement S1 à S5 sont appliquées à la feuille 40 :
- étape S1 : la feuille 40 passe sur une plaque support 403 et sa face avant (film d'aluminium 41) reçoit une couche de résine de masquage 404, de préférence une résine positive, comme illustré en figure 11 et en figure 13A,
- étape S2 : la feuille 40 étant encore sur la plaque support 403, la résine 404 est insolée à travers un masque d'insolation 405 qui comprend des ouvertures aux emplacements où de la matière métallique doit être retirée du film d'aluminium 41,
- étape S3 : la feuille 40 passe dans un bac 406 comprenant un solvant sélectif qui dissout la résine 404 dans les régions ayant été insolées, pour obtenir des ouvertures 404' représentées en figure 13B,
- étape S4 : la feuille 40 passe dans un bac 407 comprenant un solvant sélectif qui dissout le film d'aluminium 41 dans les zones où s'étendent les ouvertures 404', pour obtenir des zones 43 sans matière métallique, représentées en figure 13C,
- étape S5 : la feuille 40 passe dans un bac 408 comprenant un solvant sélectif qui dissout le masque de résine 404, comme représenté en figure 13D.

Ces étapes sont conduites en parallèle sur différentes parties de la feuille 40 qui sont de préférence de même longueur. L'étape la plus longue détermine le temps à respecter entre deux déroulements de la feuille 40 à partir du rouleau 401 et enroulements correspondants de la feuille sur le rouleau 402. Des étapes supplémentaires de rinçage et de séchage de la feuille 40, non représentées, peuvent être prévues.

La figure 12 représente le rouleau final 402, une partie de la feuille 40 étant déroulée pour montrer le résultat obtenu grâce au second procédé. La feuille 40 présente, de façon répétitive, des zones où a été formé un motif d'antenne, ici le motif d'antenne en forme de bobine 90 décrit plus haut. La distance entre chaque motif d'antenne est définie en fonction de la destination du rouleau (dimensions de l'objet à emballer).

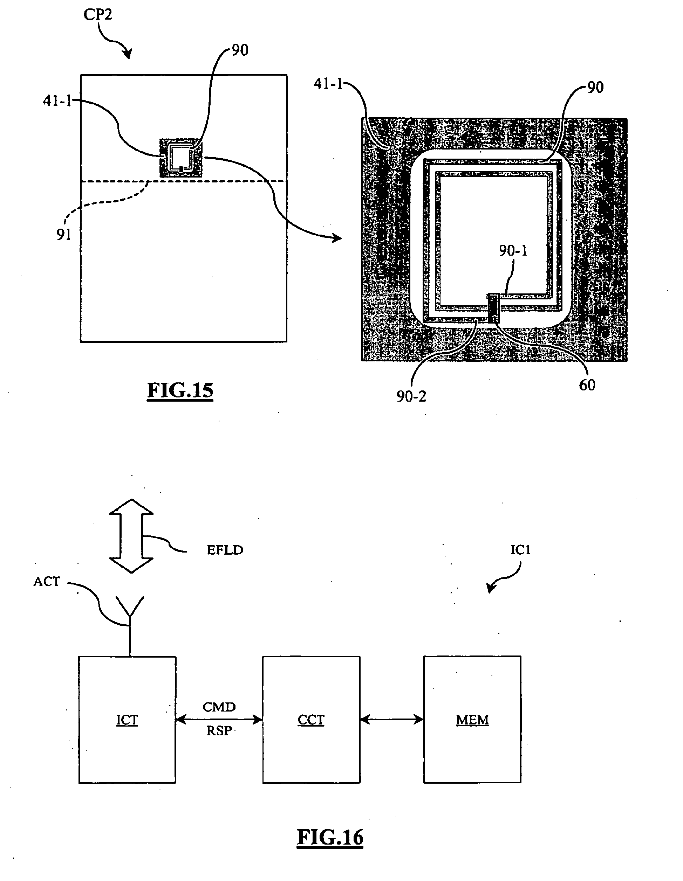

Les figures 14A et 14B illustrent une variante du second procédé. Ici, le rouleau initial 401 est formé par une feuille d'emballage 40' qui n'est pas entièrement recouverte par le film d'aluminium 41. Ce dernier forme des régions métallisées 41-1, 41-2... qui sont ainsi isolées les unes des autres en raison de l'absence de matière métallique entre ces régions. Le film d'aluminium 41 est porté par un film plastique 42' qui est de préférence plus épais que le film de liaison 42 utilisé pour réaliser les blisters. Le rouleau final 402, représenté en figure 14B, présente comme précédemment des motifs de bobines d'antennes 90 mais ceux-ci sont réalisés par retrait d'aluminium dans les régions 41-1, 41-2 uniquement. Un paquet de cigarettes CP2 emballé au moyen de la feuille 40' est représenté sur la figure 15. Un circuit intégré 60 est fixé à la bobine d'antenne 90 selon la méthode décrite plus haut (Cf. fig. 9B).

La figure 16 représente sous forme de blocs, à titre d'exemple, l'architecture classique d'un circuit intégré sans contact IC1. Le circuit intégré comprend un circuit d'antenne comportant une antenne ACT, ici une antenne de type UHF, un circuit d'interface sans contact ICT connecté à l'antenne ACT, un circuit de contrôle CCT, à logique câblée ou à microprocesseur, et une mémoire MEM, par exemple une mémoire de type effaçable et programmable électriquement (EEPROM). Le circuit de contrôle CCT reçoit via le circuit d'interface ICT des commandes CMD de lecture ou d'écriture de la mémoire, ainsi que des commandes anticollision et éventuellement des commandes d'authentification (s'il est équipé d'une fonction de cryptographie). Le circuit d'interface ICT comprend un circuit de démodulation d'un signal d'antenne induit dans l'antenne ACT par un champ électrique (ou par un champ magnétique quand l'antenne est de type bobine), et un circuit de décodage de données issues du circuit d'antenne. Le circuit ICT comprend également un circuit de modulation de l'impédance du circuit d'antenne. Le circuit d'antenne comprend l'antenne ACT et d'autres composants (capacités et/ou selfs) non représentés sur la figure, pouvant être intégrés dans la microplaquette de silicium portant le circuit intégré ou être agencés à l'extérieur de celle-ci.

Il apparaîtra clairement à l'homme de l'art que la présente invention est susceptible de diverses autres variantes de réalisation. Par exemple, un plus grand nombre d'antennes peut être prévu sur la feuille d'emballage afin que le produit emballé présente des antennes sur plusieurs de ses faces et/ou plusieurs antennes sur l'une des ses faces. Les procédé de retrait de matière métallique par action mécanique ou chimique précédemment décrits permettent de réaliser des motifs conducteurs de forme complexe et permettent par exemple de réaliser le point de connexion du circuit intégré à un endroit différent de celui où se trouve l'antenne. Ainsi, des plages de connexion de la puce peuvent être prévues en un emplacement différent de celui où se trouve l'antenne, et être reliées à l'antenne par des conducteurs formés dans le film d'aluminium de la même manière que l'antenne est formée, soit en enlevant de la matière métallique. Cela permet par exemple de monter le circuit intégré sur une face de l'objet emballé différente de celle recevant l'antenne, afin que la puce ne soit pas visible lorsque le produit est présenté à la vente. Cela permet également de mettre hors service l'étiquette électronique en prévoyant une ligne de prédécoupe de l'emballage passant par le conducteur reliant l'antenne au circuit intégré.

La présente invention est également susceptible de diverses applications et s'applique de façon générale à toute feuille souple, semi-rigide voire rigide, d'emballage, de conditionnement ou de transport de produits, comportant un film électriquement conducteur, par exemple les barquettes pour matière consommable, les sacs de transport d'objet divers, les sacs de transport de nourriture, notamment les sacs pour le transport de nourriture surgelée, les étiquettes publicitaires comprenant des parties métalliques, les bouteilles comprenant un film métallique (jus de fuit frais, lait), etc.. Les films d'aluminium étant fréquemment utilisés dans l'industrie alimentaire, ils permettent de réaliser, à moindre coût, des antennes selon l'invention. De plus, l'invention peut s'appliquer à tout type de feuille d'emballage ou de conditionnement comprenant un film électriquement conducteur initialement prévu pour des raisons autres que la réalisation d'une antenne RFID, par exemple des couches conductrices de protection antistatique, des films en cuivre, en poudre métallique agglomérée, en alliage, etc. Bien que certaines de ces applications ne permettent pas de réaliser une antenne qui soit aisément destructible par l'utilisateur, elles entrent dans le cadre de la présente invention, la réalisation d'une antenne destructible n'étant qu'un objectif optionnel de l'invention concernant certaines catégories d'applications.

Dans les exemples qui précèdent et dans les revendications, le film conducteur n'est pas recouvert sur l'une des ses faces par un film électriquement isolant, ce qui permet de réaliser l'antenne selon l'invention puis d'y connecter le circuit intégré sans contact. Ainsi, la caractéristique selon laquelle le film métallique n'est pas recouvert par un film électriquement isolant signifie que le film métallique utilisé par l'invention n'est pas intégralement pris dans un sandwich de matériaux diélectriques, notamment au moment où l'antenne est réalisée, mais n'exclut pas que l'on dispose ensuite un isolant de protection sur le circuit intégré et/ou sur l'antenne, comme cela est le cas avec la couche protectrice 64 disposée sur l'interposeur décrit plus haut en relation avec la figure 6.

## Revendications

1. Procédé de fabrication d'une étiquette électronique destinée à être apposée sur un produit (BL2, ) , comprenant les étapes consistant à :
faire apparaître, dans un film électriquement conducteur (41) d'une feuille (40) d'emballage, de conditionnement ou de transport du produit, des zones (43a, 43b, 43c) dépourvues de matière conductrice délimitant dans le film électriquement conducteur au moins un motif d'antenne formant une antenne (50a, 50b, 90) pour étiquette RFID, le film électriquement conducteur n'étant pas recouvert sur l'une des ses faces par un film électriquement isolant, et
- connecter à l'antenne (50a, 50b, 90) une puce (60) en matériau semi-conducteur formant avec l'antenne une étiquette électronique,
**caractérisé en ce qu'**il comprend une étape préalable consistant à prévoir des gorges ou des sillons (70a, 70b, 70c) sur une face du produit (BL2) dans les zones devant être dépourvues de matière conductrice, et **en ce que** l'étape consistant à faire apparaître les zones dépourvues de matière conductrice est conduite après application de la feuille d'emballage, de conditionnement ou de transport (40) sur le produit (BL2), par découpe ou abrasion du film électriquement conducteur s'étendant en face des gorges ou des sillons.

2. Procédé selon la revendication 1, dans lequel l'étape consistant à faire apparaître les zones dépourvues de matière conductrice est conduite au moyen d'un outil (80) comprenant au moins une molette (81a, 81b) de découpe ou d'abrasion pénétrant dans les gorges ou les sillons.

3. Procédé selon l'une des revendications 1 et 2, dans lequel la feuille (40) d'emballage, de conditionnement ou de transport comprend au moins un film support (42) électriquement isolant recouvert en tout ou en partie par le film électriquement conducteur (41).

4. Procédé selon l'une des revendications 1 à 3, dans lequel le produit (BL2) est une plaquette (30, 300) de type blister et la feuille d'emballage, de conditionnement ou de transport est appliquée sur une face de la plaquette (30, 300) avec le film électriquement conducteur (41) orienté vers l'extérieur.

5. Procédé selon l'une des revendications 1 à 4, dans lequel le film électriquement conducteur est un film métallique.

6. Procédé de fabrication d'un emballage de type blister (BL2, BL3), comprenant :
- une étape de fabrication d'une plaquette (30, 300) en matière plastique comprenant des alvéoles (31),
- une étape de fabrication d'une feuille d'emballage (40, ) comprenant au moins un film support (42) électriquement isolant et un film métallique (41 ) recouvrant tout ou partie du film support (42 ), et
- une étape de fermeture des alvéoles (31) en appliquant la feuille d'emballage (40) sur la plaquette (30, 300) avec le film métallique (41) orienté vers l'extérieur,
- une étape consistant à faire apparaître dans le film métallique des zones (43a, 43b, 43c) dépourvues de matière métallique délimitant dans le film métallique au moins un motif d'antenne formant une antenne (50a, 50b, 90) pour étiquette RFID,
**caractérisé en ce qu'**il comprend une étape préalable consistant à prévoir des gorges ou des sillons (70a, 70b, 70c) sur la plaquette dans les zones devant être dépourvues de matière métallique, et **en ce que** l'étape consistant à faire apparaître les zones dépourvues de matière métallique est conduite après application de la feuille d'emballage sur la plaquette, par découpe ou abrasion du film métallique s'étendant en face des gorges ou des sillons.

7. Procédé selon la revendication 6, dans lequel l'étape consistant à faire apparaître les zones dépourvues de matiére métallique est conduite au moyen d'un outil (80) comprenant au moins une molette (81a, 81b) de découpe ou d'abrasion pénétrant dans les gorges ou les sillons.

8. Procédé selon l'une des revendications 6 et 7, comprenant une étape consistant à connecter à l'antenne (50a, 50b, 90) une puce (60) en matériau semi-conducteur pour former une étiquette électronique.

## Claims

1. Method for manufacturing an electronic tag intended for being placed onto a product (BL2), comprising:
- making appear, in an electrically conducting film (41), a sheet (40) for packaging, conditioning or transporting the product, areas (43a, 43b, 43c) without conducting material delineating in the electrically conducting film at least one antenna pattern forming an antenna (50a, 50b, 90) for RFID tag, the electrically conducting film not being covered on one of its faces by an electrically insulating film, and
- connecting to the antenna (50a, 50b, 90) a chip (60) in a semiconductor material forming with the antenna an electronic tag,
**characterised in that** it comprises previously providing grooves or furrows (70a, 70b, 70c) on a face of the product (BL2) in the areas which may be without conducting material, and **in that** making the areas without conducting material appear is performed after applying the packaging, conditioning or transporting sheet (40) onto the product (BL2), by cutting or abrasion of the electrically conducting film spreading in front of the grooves of furrows.

2. Method according to claim 1, wherein making the areas without conducting material appear is performed by means of a tool (80) comprising at least a cutting or abrasion wheel (81a, 81b) penetrating the grooves or furrows.

3. Method according to one of claims 1 and 2, wherein the packaging, conditioning or transporting sheet (40) comprises at least one electrically insulating support film (42) partially or totally covered by the electrically conducting film (41).

4. Method according to one of claims 1 to 3, wherein the product (BL2) is a pack (30, 300) of blister type and the packaging, conditioning or transporting sheet is applied onto a face of the pack (30, 300) with the electrically conducting film (41) directed toward the exterior.

5. Method according to one of claims 1 to 4, wherein the electrically conducting film is a metal film.

6. Method for manufacturing a package of blister type (BL2, BL3), comprising:
- manufacturing a pack (30, 300) in plastic comprising cells (31),
- manufacturing a packaging sheet (40) comprising at least one electrically insulating support film (42) and a metal film (41) partially or totally covering the support film (42), and
- closing the cells (31) by applying the packaging sheet (40) onto the pack (30, 300) with the metal film (41) directed toward the exterior,
- making appear in the metal film, areas (43a, 43b, 43c) without metal material delineating in the metal film at least one antenna pattern forming an antenna (50a, 50b, 90) for RFID tag,
**characterised in that** it comprises previously providing grooves or furrows (70a, 70b, 70c) on the pack in the areas which may be without metallic material, and **in that** making the areas without metallic material appear is performed after applying the packaging sheet onto the pack, by cutting or abrasion of the metallic film spreading in front of the grooves or furrows.

7. Method according to claim 6, wherein making the areas without metallic material appear is performed by means of a tool (80) comprising at least a cutting or abrasion wheel (81a, 81b) penetrating the grooves or furrows.

8. Method according to one of claims 6 and 7, comprising connecting to the antenna (50a, 50b, 90) a chip (60) in a semiconductor material to form an electronic tag.

## Patentansprüche

1. Verfahren zur Anfertigung eines elektronischen Etiketts, das dazu vorgesehen ist, auf einem Produkt (BL2) angebracht zu werden, wobei es die Schritte, die folgendes beinhalten, umfasst:
- Sichtbarmachen in einem elektrisch leitenden Film (41) eines Verpackungs-, Konditionierungs- oder Transportbogens (40) des Produktes, von Zonen (43a, 43b, 43c) ohne leitendes Material, die im elektrisch leitenden Film zumindest ein Antennenmuster, das eine Antenne (50a, 50b, 90) für RFID-Etikett bildet, abgrenzen, wobei der elektrisch leitende Film auf einer seiner Seiten nicht mit einem elektrisch isolierendem Film überdeckt ist, und
- Anschließen an die Antenne (50a, 50b, 90) eines Chips (60) aus halbleitendem Material, der mit der Antenne ein elektronisches Etikett bildet,
**dadurch gekennzeichnet, dass** es einen vorherigen Schritt umfasst, der darin besteht, Auskehlungen oder Furchen (70a, 70b, 70c) auf einer Seite des Produktes (BL2) in den Zonen, die ohne leitendes Material sein sollen, vorzusehen, und **dadurch**, dass der Schritt des Sichtbarmachens der Zonen ohne leitendes Material nach Anbringen des Verpackungs-, Konditionierungs- oder Transportbogens (40) auf das Produkt (BL2) durch Schneiden oder Ausschleifen des elektrisch leitenden Films, der sich gegenüber den Auskehlungen oder Furchen erstreckt, ausgeführt wird.

2. Verfahren nach Anspruch 1, in dem der Schritt des Sichtbarmachens der Zonen ohne leitendes Material mit Hilfe eines Werkzeuges (80), das zumindest ein Rädchen (81a, 81b) zum Schneiden oder Ausschleifen, das in die Auskehlungen oder Furchen eindringt, umfasst, ausgeführt wird.

3. Verfahren nach einem der Ansprüche 1 und 2, in dem der Verpackungs-, Konditionierungs- oder Transportbogen (40) zumindest einen elektrisch isolierenden Trägerfilm (42), der ganz oder teilweise vom elektrisch leitenden Film (41) überdeckt wird, umfasst.

4. Verfahren nach einem der Ansprüche 1 bis 3, in dem das Produkt (BL2) ein Plättchen (30, 300) des Typs Blister ist und der Verpackungs-, Konditionierungs- oder Transportbogen auf einer Seite des Plättchens (30, 300) mit dem nach außen zeigenden elektrisch leitenden Film (41) angebracht wird.

5. Verfahren nach einem der Ansprüche 1 bis 4, in dem der elektrisch leitende Film ein Metallfilm ist.

6. Verfahren zur Anfertigung einer Verpackung des Typs Blister (BL2, BL3), das folgendes umfasst:
- einen Schritt zur Anfertigung eines Plättchens (30, 300) aus Kunststoff, das Zellen (31) umfasst,
- einen Schritt zur Anfertigung eines Verpackungsbogens (40), der zumindest einen elektrisch isolierenden Trägerfilm (42) und einen Metallfilm (41), der den Trägerfilm (42) ganz oder teilweise überdeckt, umfasst, und
- einen Schritt des Schließens der Zellen (31) durch Anbringen des Verpackungsbogens (40) auf die Plättchen (30, 300) mit dem nach außen zeigenden Metallfilm (41),
- einen Schritt des Sichtbarmachens im Metallfilm von Zonen (43a, 43b, 43c) ohne Metallmaterial, die im Metallfilm zumindest ein Antennenmuster, das eine Antenne (50a, 50b, 90) für RFID-Etikett bildet, abgrenzen,
**dadurch gekennzeichnet, dass** er einen vorherigen Schritt umfasst, der darin besteht, Auskehlungen oder Furchen (70a, 70b, 70c) auf dem Plättchen in den Zonen, die ohne leitendes Material sein sollen, vorzusehen, und **dadurch**, dass der Schritt des Sichtbarmachens der Zonen ohne Metallmaterial nach Anbringen des Verpackungsbogens auf dem Plättchen durch Schneiden oder Ausschleifen des Metallfilms, der sich gegenüber den Auskehlungen oder Furchen erstreckt, ausgeführt wird.

7. Verfahren nach Anspruch 6, in dem der Schritt des Sichtbarmachens der Zonen ohne Metallmaterial mit Hilfe eines Werkzeuges (80), das zumindest ein Rädchen (81a, 81b) zum Schneiden oder Ausschleifen, das in die Auskehlungen oder Furchen eindringt, umfasst, ausgeführt wird.

8. Verfahren nach einem der Ansprüche 6 und 7, das einen Schritt umfasst, der darin besteht, einen Chip (60) aus halbleitendem Material an die Antenne (50a, 50b, 90) anzuschließen, um ein elektronisches Etikett zu bilden.
